# EUROPÄISCHE PATENTSCHRIFT

(11) **EP 0 491 003 B1**
(45) Veröffentlichungstag und Bekanntmachung des Hinweises auf die Patenterteilung: **01.03.1995**
(21) Anmeldenummer: 90914729.0
(22) Anmeldetag: 24.08.1990
(51) Int. Cl.: A61K 7/13

(54) **EGALISIERENDE OXIDATIONSHAARFÄRBEMITTEL**
OXIDATIVE HAIR DYE WITH LEVELLING ACTION
COLORANT D'OXYDATION POUR TEINTURE DE CHEVEUX A EFFET EGALISANT

(30) Priorität: 04.09.1989 DE 3929333
(43) Veröffentlichungstag der Anmeldung: 24.06.1992
(73) Patentinhaber: Henkel Kommanditgesellschaft auf Aktien, 40191 Düsseldorf (DE)
(72) Erfinder: MATZIK, Iduna, D-4300 Essen 15 (DE); HÖFFKES, Horst, D-4000 Düsseldorf (DE)
(86) Internationale Anmeldenummer: EP9001419
(87) Internationale Veröffentlichungsnummer: WO9103230

(56) Entgegenhaltungen:
- EP-A- 0 063 736
- DE-A- 2 942 376
- Patent abstracts of Japan, volume 8, n 222 (C-246) (1659) 9 October 1984

## Beschreibung

Die Erfindung betrifft Oxidationshaarfärbemittel, die als Entwicklerkomponente ein 2,4,5,6-Tetraaminopyrimidin und als Hilfsmittel zur Verbesserung der Gleichmäßigkeit der Haaranfärbung Histidin oder ein wasserlösliches Salz davon enthalten.

Für das Färben von Haaren spielen die sogenannten Oxidationsfarben, die durch oxidative Entwicklung aus Oxidationsfarbstoffvorprodukten gebildet werden, eine bevorzugte Rolle. Oxidationshaarfärbemittel enthalten solche Oxidationsfarbstoffvorprodukte zusammen mit Färbehilfsmitteln in einem kosmetischen Träger. Als Oxidationsfarbstoffvorprodukte werden sogenannte Entwickler bzw. Oxidationsbasen eingesetzt, die entweder untereinander oder mit sogenannten Kupplern (color modifier) unter dem Einfluß von Oxidationsmitteln die Farbstoffe ausbilden. Als Entwicklersubstanzen werden üblicherweise primäre aromatische Amine mit einer weiteren in Para- oder Orthoposition befindlichen, freien oder substituierten Amino- oder Hydroxygruppe sowie Diaminopyridine, heterocyclische Hydrazonderivate, 4-Aminopyrazolonderivate und Tetraaminopyrimidine eingesetzt. Als sogenannte Kupplersubstanzen werden m-Phenylendiamine, m-Aminophenole, Resorcine, Naphthole und Pyrazolone verwendet.

Als Färbehilfsmittel werden z. B. Antioxidantien zur Verhinderung vorzeitiger Oxidation, pH-Wert-Stellmittel, Puffersubstanzen und haarkosmetisch wirksame Zusätze verwendet. Geeignete Träger sind z. B. Cremes, Emulsionen, Gele oder auch schäumende Lösungen, Shampoos, Aerosole oder andere Zubereitungen, die für die Anwendung auf dem Haar geeignet sind.

Eine besonders wertvolle Entwicklerkomponente, die mit vielen bekannten Kupplern ein breites Spektrum intensiver Farbnuancen mit guten Echtheitseigenschaften ausbildet, ist das 2,4,5,6-Tetraaminopyrimidin und dessen Derivate. Viele der mit 2,4,5,6-Tetraaminopyrimidin erzielbaren Haarfärbungen haben den Nachteil, daß sie ungleichmäßig auf das Haar aufziehen und z. B. die stärker strapazierten Haarspitzen wesentlich intensiver anfärben als die weniger geschädigten Bereiche des Haaransatzes.

Es wurde nun gefunden, daß sich die Gleichmäßigkeit der Haaranfärbung mit Oxidationshaarfärbemitteln, die als Entwicklerkomponente 2,4,5,6-Tetraaminopyrimidin oder dessen Derivate enthalten, durch einen Zusatz von Histidin oder einem wasserlöslichen Salz davon wesentlich verbessern läßt.

Das Histidin kann in freier Form oder als Salz, z. B. als Carbonat, Hydrochlorit, Sulfat, Phosphat, Acetat, Propionat, Lactat oder Citrat eingesetzt werden. Zur Erzielung einer merklichen Egalisierung ist es ausreichend, das Histidin oder seine Salze in einer Menge von 0,1 - 10 Gew.-%, bevorzugt von 1 - 5 Gew.-%, berechnet als freies Histidin, dem Färbemittel zuzusetzen. (Alle Angaben in Gew.-% beziehen sich auf den Anteil bezogen auf das erfindungsgemäße Oxidationshaarfärbemittel vor der Zugabe eines Oxidationsmittels)
Als weiteres, die Farbtiefe und Egalisierung der Färbung verbesserndes, Hilfsmittel hat sich das N-(2-Hydroxyethyl)-acetamid erwiesen, dessen günstige Wirkung auf Intensität und Echtheit von Haarfärbungen schon aus DE 2 942 376 C2 bekannt ist. In Kombination mit dem Histidin ergibt sich aber eine überproportionale, d.h. synergistische Steigerung der Farbtiefe und Egalität der Färbung. Das 2-Hydroxyethyl-acetamid wird bevorzugt in einer Menge von 0,1 - 5 Gew.-% in die Oxidationshaarfärbemittel eingesetzt. Das als Entwicklerkomponente geeignete 2,4,5,6-Tetraaminopyrimidin ist z. B. aus DE 2 359 399 bekannt. Geeignet sind aber auch dessen Derivate der allgemeinen Formel I 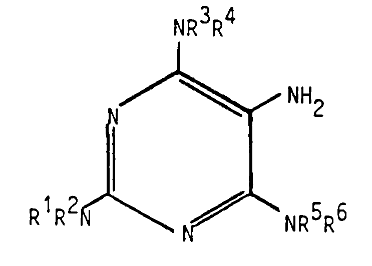
in der R¹ bis R⁶ unabhängig voneinander Wasserstoff, Alkylgruppen mit 1 - 4 C-Atomen oder Hydroxyalkylgruppen mit 2 - 4 C-Atomen oder die Gruppen R¹ und R² bzw. R³ und R⁴ bzw. R⁵ und R⁶ jeweils gemeinsam mit dem Stickstoffatom einen Morpholin-, Piperidin-, Pyrrolidin- oder Piperazinring bilden, oder deren Salze mit anorganischen oder organischen Säuren, z. B. in Form der Hydrochloride, Sulfate, Phosphate, Acetate, Propionate, Lactate oder Citrate.

Als Kuppler sind praktisch alle bekannten Kupplerverbindungen, die mit Tetraaminopyrimidin brauchbare Farben ausbilden, geeignet. Bevorzugte Kuppler für die erfindungsgemäßen Oxidationshaarfärbemittel sind aber Resorcin, Methylresorcin, 2,7-Dihydroxynaphthalin, 2,6-Dihydroxy-3,4-dimethylpyridin und deren Salze.

Die erfindungsgemäßen Haarfärbemittel können neben 2,4,5,6-Tetraaminopyrimidin und dessen Derivaten der Formel I noch andere, bekannte Entwicklersubstanzen enthalten, die zur Modifizierung der Farbnuancen und zur Erzeugung natürlich aussehender Farbtöne erforderlich sind, z. B. p-Phenylendiamin, p-Aminophenol, o-Aminophenol oder deren Derivate.

Gegebenenfalls können auch direktziehende Farbstoffe zusätzlich zur Nuancierung eingesetzt werden. Solche direktziehenden Farbstoffe sind z. B. Nitrophenylendiamine, Nitroaminophenole, Nitrophenole, Antrachinonfarbstoffe oder Indophenole.

Entwickler- und Kupplersubstanzen werden im allgemeinen in etwa molaren Mengen eingesetzt, es ist jedoch nicht nachteilig, wenn einzelne Oxidationsfarbstoffvorprodukte im Überschuß vorliegen, so daß Entwicklersubstanzen und Kupplersubstanzen in einem Molverhältnis von 1 : 0,5 bis 1 : 2 enthalten sein können. 2,4,5,6-Tetraaminopyrimidin und dessen Derivate sind in den erfindungsgemäßen Haarfärbemitteln bevorzugt in Mengen von 0,1 bis 10 Millimol pro 100 g des Haarfärbemittels einhalten. Neben den genannten Egalisierhilfsmitteln, Histidin und N-(2-Hydroxyethyl-)acetamid, können die erfindungsgemäßen Oxidationshaarfärbemittel noch weitere Färbehilfsmittel enthalten. Unter diesen sind besonders zu nennen:
- Antioxidantien, z. B. Natriumsulfit, Ascorbinsäure,
- Alkalisierungsmittel, z. B. Ammoniak, Alkanolamine wie Mono-, Di- und Triethanolamin, Isopropanolamin, basische Aminosäuren wie z. B. Ornithin und Arginin,
- Puffersubstanzen, z. B. Ammoniumcarbonat, Ammoniumchlorid, Ammoniumsulfat,
- Komplexbildner, z. B. 1-Hydroxyethan-1,1-diphosphonsäure, Nitrilotriessigsäure, Ethylendiamintetraessigsäure,
- haarkosmetische Hilfsmittel, z. B. wasserlösliche, kationische Polymere, Glucose, D-Panthenol, Proteinderivate, Cholesterin,
- Lösungsvermittler, z. B. Isopropanol, 1,2-Propylenglykol, Glycerin,
- direktziehende Haarfarbstoffe, z. B. Nitrophenylendiamine, Nitroaminophenole und Antrachinonfarbstoffe
Zur Herstellung der erfindungsgemäßen Oxidationshaarfärbemittel werden die Oxidationsfarbstoffvorprodukte und die Färbehilfsmittel in einen geeigneten kosmetischen Träger eingearbeitet. Bevorzugte Träger sind Gele und Cremeemulsionen. Übliche Bestandteile solcher Träger sind neben Wasser Seifen, insbesondere Ammoniumoleat, nichtionogene, ampholytische und anionische Netz- und Emulgiermittel, z. B. Fettalkoholsulfate, Fettalkoholpolyglykolethersulfate, Alkansulfonate, Alpha-Olefinsulfonate, Ethylenoxidanlagerungsprodukte an Fettalkohole, an Fettsäuren, an Alkylphenole, an Glycerin- und Sorbitan-Fettsäurepartialester, Fettsäurealkanolamide und Fettkomponenten, z. B. Fettalkohole mit 12 - 22 C-Atomen, Paraffinöle oder Fettsäureester, wasserlösliche Verdikkungsmittel, z. B. Methyl- oder Hydroxyethylcellulose, Stärke, Carboxymethylcellulose, Guar, Pflanzengummen und synthetische, wasserlösliche Polymerisate mit verdickender Wirkung sowie Parfumöle.

In einer besonders bevorzugten Ausführungsform stellt das erfindungsgemäße Haarfärbemittel eine Creme-Emulsion dar, die 0,1 - 25 Gew.-% einer Fettkomponente, 0,5 - 30 Gew.-% eines Emulgators aus der Gruppe der anionischen, nichtionischen, zwitterionischen oder ampholytischen Tenside, jeweils 0,1 - 10 Millimol pro 100 g Entwicklerkomponenten und Kupplerkomponenten sowie übliche Färbehilfsmittel enthält. Das Histidin ist darin in einer Menge von 0,1 - 10 Gew.-% enthalten.

Die oxidative Entwicklung der Färbung kann grundsätzlich mit Luftsauerstoff erfolgen. Bevorzugt wird jedoch kurz vor der Anwendung ein chemisches Oxidationsmittel zugesetzt, besonders dann, wenn außer der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus solchen Wasserstoffperoxidanlagerungsprodukten in Frage.

Die Anwendung der erfindungsgemäßen Haarfärbemittel kann, unabhängig von der Art der kosmetischen Zubereitung, z. B. als Creme, Gel oder Shampoo, bevorzugt in einem pH-Bereich von 6 - 10 erfolgen. Besonders bevorzugt ist die Anwendung der Haarfärbemittel in einem schwach alkalischen Milieu. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern ohne ihn jedoch hierauf zu beschränken.

### Beispiele

Es wurden erfindungsgemäße Haarfärbemittel in Form einer Haarfärbe-Cremeemulsion hergestellt. Haarsträhnen, die im unteren Teil unvorbehandelt, im oberen Teil definiert vorgeschädigt waren, damit angefärbt und die Farbabstände zwischen geschädigten und ungeschädigten Haarregionen gemessen und rechnerisch ausgewertet.

### 1. Herstellung der Haarfärbe-Cremeemulsion

Die Haarfärbe-Cremeemulsionen wurden entsprechend der in Tabelle 1 angegebenen Zusammensetzungen wie folgt hergestellt: Die Fettalkohole wurden aufgeschmolzen und mit dem Fettalkoholethersulfat und 30 g Wasser bei 90 °C emulgiert. Die Emulsion wurde bis 60 °C abgekühlt. Dann wurde die Ascorbinsäure und das Histidin in 20 g Wasser von 90 °C gelöst zugegeben. Anschließend wurden die Oxidationsfarbstoff-Vorprodukte in 10 g Wasser von 90 °C gelöst, mit konzentriertem Ammoniak auf pH = 9,5 eingestellt und zugegeben. Nach dem Abkühlen auf 30 °C wurden die übrigen Komponenten in der angegebenen Reihenfolge zugesetzt und die Emulsionen mit konzentrierter Ammoniaklösung auf pH = 9,5 eingestellt und mit Wasser auf 100 g ergänzt und homogenisiert.

Zum Vergleich wurden die Rezepturen auch ohne Histidin hergestellt (anstatt dessen wurde eine entsprechende Menge Wasser zugegeben).

### 2. Haaranfärbung

Die oxidative Entwicklung der Färbung wurde mit 6 %iger Wasserstoffperoxid-Lösung als Oxidationsmittel durchgeführt. Hierzu wurden 100 g der Emulsion mit 50 g Wasserstoffperoxidlösung (6 %ig) versetzt und vermischt. Die gebrauchsfertigen Färbezubereitungen wurden dann auf ca. 15 cm lange und ca. 2 g schwere Haarsträhnen aufgetragen, die in der folgenden Weise vorbehandelt waren:
Die obere Hälfte der Haarsträhne (der Bereich der Haarspitze) wurde 30 Minuten lang mit der wäßrigen Lösung eines Kaltwellmittels auf Basis Ammoniumthioglykolat behandelt. Nach der Fixierung (10 Minuten, Kaliumbromatlösung) wurde die gleiche Hälfte der Haarsträhnen mit einer wäßrigen Lösung aus Wasserstoffperoxid und Ammoniumperoxiddisulfat ultrablondiert. Anschließend erfolgte noch einmal eine Behandlung mit dem Kaltwellmittel, der Fixierung und der Ultrablondierung. Die untere Hälfte der Haarsträhne (Wurzel bereich) wurde nur einmal ultrablondiert. Auf diese Weise wurden zwei unterschiedlich stark strapazierte Haarsträhnen-Hälften erhalten.

Die Färbecremes wurden dann ca. 30 Minuten bei 27 °C auf den Haarsträhnen gelassen, dann mit Wasser ausgewaschen. Nach dem Ausspülen mit Wasser wurden die Strähnchen dann getrocknet.

### 3. Ermittlung der Gleichmäßigkeit der Haaranfärbung durch Messung der Farbabstands-Werte (DE-Werte)

Jede Haarsträhne wurde an acht Stellen (vier im Bereich des Haaransatzes und vier im Bereich der Haarspitze) mit Hilfe eines Farbmeßsystems der Firma Datacolor vermessen. Dabei wurde die zu vermessende Probe in einer Einspannvorrichtung am Spektralphotometer fixiert und die Remissionswerte über den Bereich des sichtbaren Lichtes von 390 - 700 nm im Abstand von 10 nm gemessen und über einen Rechner (Minicomputer HP 2113 E) verarbeitet. Das Rechnerprogramm ermittelt die Normfarbwerte nach dem CIE-System (Commission Internationale de l'Eclairage) entsprechend DIN 5033 und rechnet sie in Farbabstandszahlen nach DIN 6174 um.

In der Praxis der Haaranfärbung mit Oxidationshaarfärbemitteln können DE-Werte unterhalb 12 als befriedigend und akzeptabel bewertet werden, wenn kein, mit bloßem Auge sichtbarer, Farbton-Unterschied besteht.

Die mit den erfindungsgemäßen Haarfärbemitteln der Beispiele 1 - 5 angefärbten Haarsträhnen zeigten zwischen der stärker geschädigten oberen Hälfte und dem weniger geschädigten unteren Bereich der Haare keinen, mit bloßem Auge erkennbaren Farbton-Unterschied. Die sichtbaren Unterschiede betreffen lediglich die Intensität bzw. Farbstärke.

**Tabelle 1**

| Zusammensetzung in g/100 g | 1 | 2 | 3 | 4 | 5 |
|---|---|---|---|---|---|
| Hydr. Talgfettalkohol C_{16/18} | 6,5 | 6,5 | 6,5 | 6,5 | 6,5 |
| Hydr. Kokosfettalkohol C_{12/18} | 2,0 | 2,0 | 2,0 | 2,0 | 2,0 |
| Fettalkohol C_{12/14} + 2 EO-sulfat Na-Salz, 28 %ige wäßrige Lösung | 25,0 | 25,0 | 25,0 | 25,0 | 25,0 |
| Wasser | 30,0 | 30,0 | 30,0 | 30,0 | 30,0 |
| Histidin | 5,0 | 1,0 | 1,0 | 1,0 | 1,0 |
| Ascorbinsäure | 0,4 | 0,4 | 0,4 | 0,4 | 0,4 |
| Wasser | 20,0 | 20,0 | 20,0 | 20,0 | 20,0 |
| 2,4,5,6-Tetraaminopyrimidin | 2,082 | 2,082 | 2,082 | 2,082 | 2,082 |
| Resorcin | - | - | - | 0,220 | 0,220 |
| 2-Methylresorcin | 0,993 | 0,496 | 0,496 | - | - |
| 2,7-Dihydroxy-3,4-dimethyl-pyridin | - | 0,492 | 0,492 | 0,740 | 0,740 |
| (NH₄)₂ CO₃ | 1,0 | 1,0 | 1,0 | 1,0 | 1,0 |
| Na₂SO₃ | 0,5 | 0,5 | 0,5 | 0,5 | 0,5 |
| 2-Hydroxyethylacetamid | - | - | 0.5 | - | 0,5 |
| konz. Ammoniak-Lösung | bis pH = 9,5 | | | | |
| Wasser | ad 100 g | | | | |
| Färbenuancen | mahagoni | korallrot | korallrot | henna-gold | henna-gold |
| DE-Wert (Farbabstand) | 4,48 | 10,77 | 8,56 | 10,88 | 7,22 |
| Vergleich DE-Wert ohne Histidin | 20,01 | 27,23 | 27,23 | 25,56 | 25,56 |

## Patentansprüche

1. Oxidationshaarfärbemittel, enthaltend Oxidationsfarbstoffvorprodukte und Färbehilfsmittel in einem kosmetischen Träger, wobei als Entwicklerkomponente wenigstens ein 2,4,5,6-Tetraaminopyrimidin oder ein Derivat davon oder deren wasserlöslichen Salze enthalten sind, dadurch gekennzeichnet, daß als Egalisierhilfsmittel Histidin oder ein wasserlösliches Salz davon enthalten ist.

2. Oxidationshaarfärbemittel nach Patentanspruch 1, dadurch gekennzeichnet, daß als weiteres Färbehilfsmittel N-2-hydroxyethyl-acetamid enthalten ist.

3. Oxidationshaarfärbemittel nach Patentanspruch 1, dadurch gekennzeichnet, daß Histidin oder dessen Salze in einer Menge von 0,1 - 10 Gew.-%, berechnet als freies Histidin enthalten sind.

4. Oxidationshaarfärbemittel nach Patentanspruch 2, dadurch gekennzeichnet, daß N-2-hydroxyethyl-acetamid in einer Menge von 0,1 - 5 Gew.-% enthalten ist.

5. Oxidationshaarfärbemittel nach Patentanspruch 1 oder 2, dadurch gekennzeichnet, daß 2,4,5,6-Tetraaminopyrimidin oder dessen Derivate mit der Formel 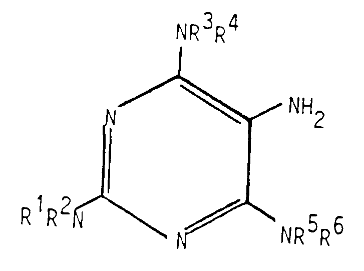 in der R¹ bis R⁶ unabhängig voneinander Wasserstoff, Alkylgruppen mit 1 - 4 C-Atomen oder Hydroxyalkylgruppen mit 2 - 4 C-Atomen oder die Gruppen R¹ und R³ bzw. R³ und R⁴ bzw. R⁵ und R⁶ jeweils gemeinsam mit dem Stickstoffatom einen Morpholin-, Piperidin-, Pyrrolidin- oder Piperazinring bilden, oder deren Salze und als Kupplerkomponenten eine oder mehrere Verbindungen aus der Gruppe Resorcin, 2-Methylresorcin, 2,7-Dihydroxynaphthalin, 2,6-Dihydroxy-3,4-dimethylpyridin oder deren Salze enthalten sind.

## Claims

1. Oxidation hair dyes containing oxidation dye precursors and dyeing auxiliaries in a cosmetic carrier, at least one 2,4,5,6-tetraaminopyrimidine or a derivative thereof or water-soluble salts thereof being present as the developer component, characterized in that histidine or a water-soluble salt thereof is present as a levelling agent.

2. Oxidation hair dyes as claimed in claim 1, characterized in that N-2-hydroxyethyl acetamide is present as another dyeing auxiliary.

3. Oxidation hair dyes as claimed in claim 1, characterized in that histidine or a salt thereof is present in a quantity of 0.1 to 10% by weight, expressed as free histidine.

4. Oxidation hair dyes as claimed in claim 2, characterized in that N-2-hydroxyethyl acetamide is present in a quantity of 0.1 to 5% by weight.

5. Oxidation hair dyes as claimed in claim 1 or 2, characterized in that they contain 2,4,5,6-tetraaminopyrimidine or derivatives thereof corresponding to the following formula 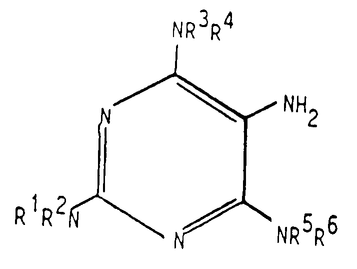 in which R¹ to R⁶ independently of one another represent hydrogen, C₁₋₄ alkyl groups or C₂₋₄ hydroxyalkyl groups or the substituents R¹ and R² or R³ and R⁴ or R⁵ and R⁶ together with the nitrogen atom form a morpholine, piperidine, pyrrolidine or piperazine ring, or salts thereof and, as coupler components, one or more compounds from the group consisting of resorcinol, 2-methyl resorcinol, 2,7-dihydroxynaphthalene, 2,6-dihydroxy-3,5-dimethyl pyridine or salts thereof.

## Revendications

1. Teinture pour cheveux par oxydation contenant des produits précurseurs de colorants par oxydation et des adjuvants de couleur dans un support cosmétique, dans laquelle comme composant de développeur, au moins une 2,4,5,6-tétraaminopyrimidine ou un de ses dérivés ou de ses sels solubles dans l'eau y sont contenus, caractérisée en ce que comme agent d'homogénéisation de l'histidine ou un de ses sels solubles dans l'eau y est contenu.

2. Teinture pour cheveux par oxydation selon la revendication 1, caractérisée en ce que comme adjuvant de couleur supplémentaire le N-(2-hydroxyéthyl)-acétamide y est contenu.

3. Teinture pour cheveux par oxydation selon la revendication 1, caractérisée en ce que l'histidine ou ses sels y sont contenus en une quantité allant de 0,1 à 10 % en poids, calculé en tant qu'histidine libre.

4. Teinture pour cheveux par oxydation selon la revendication 2, caractérisée en ce que le N-(2-hydroxyéthyl)-acétamide y est contenu en une quantité allant de 0,1 à 5 % en poids.

5. Teinture pour cheveux par oxydation selon la revendication 1 ou la revendication 2, caractérisée en ce que la 2,4,5,6-tétraaminopyrimidine ou ses dérivés de formule 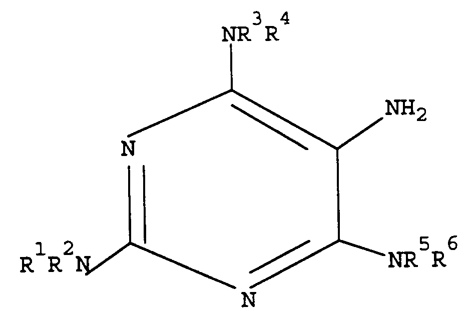 dans laquelle R¹ à R⁶ indépendamment les uns des autres, sont de l'hydrogène, des radicaux alcoyle ayant de 1 à 4 atomes de carbone ou des groupes hydroxyalcoyle ayant de 2 à 4 atomes de carbone ou bien les radicaux R¹ et R² ou R³ et R⁴ ou R⁵ et R⁶ respectivement, ensemble avec l'atome d'azote forment un cycle morpholine, pipéridine, pyrrolidine ou piperazine, ou leurs sels et comme composants de couplage un ou plusieurs composés choisis dans le groupe du resorcinol, du 2-méthylrésorcinol, du 2,7-dihydrorynaphtalène, de la 2,6-dihydroxy- 3,4-diméthylpy-ridine ou de leurs sels, y sont inclus.
